# FASCICULE DE BREVET EUROPEEN

(11) **EP 2 243 461 B1**
(45) Date de publication et mention de la délivrance du brevet: **16.10.2013**
(21) Numéro de dépôt: 10004277.9
(22) Date de dépôt: 22.04.2010
(51) Int. Cl.: G06F 19/00, A61J 7/04, A61J 1/03

(54) **Dispositif de mesure de l'observance du suivi d'un médicament sous blister**
Messvorrichtung zur Beobachtung der Nachverfolgung eines Medikaments in einer Blisterverpackung
Device for measuring observance of the follow-up of a drug in a blister pack

(30) Priorité: 23.04.2009 FR 0901990
(43) Date de publication de la demande: 27.10.2010
(73) Titulaire: ABR Pharma Société par actions simplifiée, 75002 Paris (FR)
(72) Inventeur: Bousquet, Bernard, 75005 Paris (FR)
(74) Mandataire: Benech, Frédéric

(56) Documents cités:
- EP-A- 1 536 470
- WO-A-2004/002396
- WO-A2-2009/087313
- US-A1- 2004 178 112
- US-A1- 2005 063 102
- US-A1- 2007 084 746

## Description

La présente invention concerne un dispositif de mesure de l'observance de la prise de médicaments, du type comprenant une plaquette équipée d'un circuit électronique de détection et d'enregistrement du moment (date et heure) d'extraction de pilules appartenant à un blister.

Elle concerne également un procédé de mesure de l'observance utilisant un tel dispositif.

Outre ses applications en recherche clinique, elle trouve surtout une application particulièrement importante, bien que non exclusive, dans le domaine de la mesure et de l'amélioration de l'usage des médicaments de prescription, permettant de mesurer l'observance, c'est à dire le degré de respect par des patients ambulatoires d'une prescription médicamenteuse faite par un médecin, de ville ou hospitalier, puis délivrée par un pharmacien, d'officine le plus souvent, ou hospitalier en particulier dans le cas de médicaments qui réglementairement ne peuvent pas être distribués en pharmacie de ville.

Par blisters (dénomination anglo-saxonne désormais usuelle en français) on entend des plaquettes thermoformées munies d'alvéoles contenant des pilules c'est-à-dire au sens large des unités thérapeutiques solides, comprimés, gélules... , lesdites alvéoles ayant leur orifice fermé par une feuille ou un film (en général en aluminium). Ce film de protection se rompt plus ou moins facilement quand l'utilisateur extrait la pilule, le plus souvent en la poussant, pour forcer son passage au travers du film de protection.

On connaît déjà des procédés et dispositifs permettant d'enregistrer des données historiques sur les prises médicamenteuses par un patient. Recourrant à des technologies différentes, ils revendiquent une utilité, en recherche clinique, dans le cadre des études expérimentales permettant d'évaluer l'efficacité et la tolérance d'un médicament et nécessaires à l'obtention d'une Autorisation de Mise sur le Marché (AMM).

De tels procédés, non adaptés à un usage de routine en pratique quotidienne, sont destinés au cadre expérimental, c'est-à-dire à la recherche clinique, où le médicament est remis ou donné au patient par le médecin investigateur sans passer par une prescription préalable décidée et réalisée par un médecin puis suivie par une délivrance en pharmacie.

On connaît ainsi une solution du type mentionné ci-dessus, constituée par un blister encarté entre deux couches de carton, ou autre protection encombrante et rigide, dans laquelle est intégré au moment de la fabrication du lot thérapeutique un circuit électronique de détection.

Un tel dispositif porte toute une série de références propres à l'expérimentation dont il fait partie. Chaque pilule doit de plus correspondre à une entrée dans le microprocesseur, ce qui rend la partie électronique lourde et trop coûteuse pour un usage unique.

Cette solution présente de ce fait une utilisation limitée.

Elle est de plus complexe et coûteuse à fabriquer, ne s'adapte pas aisément à toutes les tailles de pilules et aux multiples contraintes réglementaires, d'ergonomie. En particulier, si elle répond aux contraintes des conditionnements pour lesquels une certaine résistance à l'extraction est souhaitée, par exemple pour les médicaments à usage pédiatrique, elles ne s'adaptent pas aux cas où une ouverture facile est souhaitée. C'est par exemple le cas pour les patients ayant une vision et/ou une motricité diminuées, des troubles cognitifs non forcément identifiés, etc.

Une autre solution connue utilise un circuit électrique réalisé par l'impression d'encres aqueuses sur du papier. Ce circuit électrique est alors entièrement dissocié de la partie électronique. Le circuit de la partie électronique ne peut en effet pas être en papier en raison de la nécessité de soudure.

Une telle solution impose dès lors une étape de fabrication supplémentaire. Elle est de plus fragile, pose des problèmes de fiabilité des encres en terme de maintien dans le temps d'une bonne conductibilité et de précision concernant leur résistivité. Elle impose de ce fait un procédé de contrôle-qualité nécessairement complexe et nécessite une augmentation de la taille ainsi qu'une rigidification d'un support nécessairement opaque pour améliorer sa fiabilité, ce qui est incompatible avec un usage du médicament en conditions usuelles.

Cette solution a enfin nécessairement pour finalité un usage expérimental exclusif, c'est à dire réservé à la recherche clinique en raison de la technologie utilisée.

On connaît enfin des solutions répondant mieux (en apparence) au problème posé que les solutions précédentes, car présentant des tentatives d'utilisation dans un contexte de médicaments de prescription.

Il s'agit de flacons ou bouteilles avec bouchon électronique, agencés pour enregistrer chaque ouverture.

De tels dispositifs présentent cependant, ici encore, des inconvénients, car ils imposent le déconditionnement du médicament, c'est-à-dire la sortie de toutes les pilules de leurs alvéoles protectrices avant reconditionnement dans un flacon.

Il nécessite donc soit l'intervention d'un tiers pour effectuer ces opérations, soit l'intervention du patient.

Dans les deux cas, cette solution n'est pas satisfaisante.

L'intervention d'un tiers impose des autorisations administratives préalables souvent impossibles à obtenir hors d'un cadre purement d'expérimentation ou de pilote.

Quant à l'intervention du patient, il s'agit d'une contrainte encore plus inacceptable pour les agences sanitaires, seul l'usage dans un contexte de recherche clinique étant autorisé sur le plan réglementaire.

Il existe de plus un facteur de confusion inhérent au dispositif formé par un flacon et son bouchon enregistreur, qui rend l'interprétation secondaire des données difficiles dans la mesure où l'on ne sait jamais si l'enregistrement d'une ouverture du flacon correspond à une prise ou à un remplissage.

Le système enregistre en effet l'ouverture du flacon ce qui n'équivaut pas obligatoirement à l'authentification d'une sortie de pilule, puisqu'on peut très bien ne pas extraire de pilule ou en extraire plusieurs à cette occasion.

Les patients apprécient par ailleurs peu de transporter et manipuler un flacon jugé encombrant, pour une prise à l'extérieur, alors qu'ils ont pris l'habitude d'utiliser des blisters dont l'usage peut être très discret.

Enfin, l'opération de déconditionnement-reconditionnement conduit à modifier l'aspect d'un médicament commercialisé sous blister ce qui impose la réalisation préalable, quand elle est possible, d'une étude de stabilité du médicament toujours coûteuse.

La présente invention vise à palier les inconvénients de l'art antérieur et à proposer un dispositif répondant mieux que ceux antérieurement connus aux exigences de la pratique, en particulier hors du domaine de la recherche clinique, pour des produits de prescription délivrés par un pharmacien à des patients ambulatoires classiques, en ce qu'elle est peu coûteuse, peu contraignante pour lesdits patients, car d'utilisation facile et intuitive, et ce de façon fiable car n'engendrant pratiquement aucun risque de faux positifs ou de faux négatifs, et ce quelque soit les dimensions et/ou les formes des blisters et/ou des pilules. Elle est indépendante de l'habilité du manipulateur, qui doit pouvoir être un patient à la vision non totalement corrigée et/ou présentant un trouble cognitif non diagnostiqué, ou encore des troubles articulaires, moteur, ou neurologiques...

L'invention part notamment du principe d'utiliser autant de micro-interrupteurs (ou groupe d'interrupteurs) que d'unités thérapeutiques, mais avec une défaillance zéro ou sensiblement zéro, et ce en utilisant les blisters du commerce. Il est donc proposé des interrupteurs irréversibles et fiables, c'est-à-dire tels que l'ouverture d'un interrupteur corresponde effectivement à la sortie complète et évidente d'une unité thérapeutique hors de son alvéole protectrice, pour tous types de médicaments.

Chaque dispositif comporte les mêmes configurations d'alvéoles que le blister du médicament auquel il correspond et peut être vendu à part indépendamment des blisters.

Autrement dit l'invention permet ainsi d'éviter de générer des données erronées avec les blisters du commerce.

Pour ce faire il faut d'une part que chaque interrupteur s'ouvre de façon irréversible, car si un interrupteur se referme, il empêche la comptabilisation de l'ouverture ultérieure d'un autre interrupteur et d'autre part que tout faux contact puisse être pratiquement éliminé, surtout du à la pellicule d'aluminium.

Par ailleurs, il est essentiel que l'ouverture d'un interrupteur soit totalement concomitante à l'extraction d'une pilule, tout en ne nécessitant aucun effort perturbant supplémentaire de la part du patient.

En effet, dans le cas contraire, l'utilisateur multipliera les tentatives d'extraction. La résultante sera alors la multiplication de faux-positifs, le système enregistrant des ouvertures successives et rapprochées d'interrupteurs alors que le patient n'a en fait extrait qu'une seule pilule (ou aucune).

Ce risque est renforcé notamment dans le cas de blisters réalisés à partir de matériaux opaque, car aucun contrôle visuel par le patient n'est possible pour vérifier la présence ou l'absence d'une pilule dans son alvéole.

De même, les interrupteurs ne doivent pas s'ouvrir spontanément en cas de manipulation non précautionneuse du blister. En particulier l'ouverture d'un interrupteur liée à l'extraction d'une pilule ne doit pas créer un risque d'ouverture des interrupteurs adjacents du fait du pliage et des pressions inévitablement exercés par l'utilisateur.

Avec l'invention, le patient, sans compétence ou entraînement particuliers, ayant d'éventuels déficits sensoriel, articulaire, moteur et cognitif, va ainsi pouvoir enregistrer ses prises de médicaments délivrés par le pharmacien pour des blisters du commerce, avec des contraintes au-dessous du seuil minimum admis par les autorités sanitaires.

Dans ce but, l'invention propose notamment un dispositif de mesure de l'observance comprenant une plaquette équipée d'un circuit électronique de détection et d'enregistrement du moment d'extraction des pilules appartenant à un blister et des moyens de fixation du blister à la plaquette, la plaquette étant formée d'un support comportant la partie conducteur dudit circuit pour former des interrupteurs propres à être ouverts lorsque les pilules sont extraites manuellement par un utilisateur, **caractérisé en ce que** la partie conducteur comprend un fil imprimé de métal conducteur, ledit fil comportant des portions conductrices identiques réparties régulièrement en parallèle le long d'un chemin conducteur, ledit chemin conducteur étant à chaque fois agencé pour présenter une plus grande résistance que la portion en parallèle, en ce que le support est formé d'une feuille de plastique mince, souple, pliable, d'une grande flexibilité, transparente, en ce qu'il comprend des portions frangibles découpées sur les cotés et réparties régulièrement pour correspondre à une pilule correspondante, en ce que au moins quatre points de rupture de ces portions conductrices sont ménagés par portion frangible et pilule correspondante, au bord desdites portions frangibles de sorte que la portion frangible de la plaquette et au moins un point de rupture de la portion conductrice correspondante sont rompues simultanément lorsqu'une pilule est extraite et en ce que le circuit comporte des moyens de mesure de l'impédance globale de la partie conducteur pour en déduire le moment de l'extraction d'une pilule.

Par fil ou film métallique, on entend notamment un chemin conducteur longiligne d'épaisseur faible (quelques microns ou dizaines de microns) et de largeur par exemple comprise entre 0,1 et 0,5 mm, par exemple 0,2 mm. Il est par exemple obtenu par collage/laminage sur la feuille de plastique, de façon connue en elle-même, similaire à une impression réalisée dans le cadre de la fabrication d'un circuit imprimé.

Par au bord desquels, on entend à cheval sur la périphérie c'est à dire en partie à l'intérieur de la zone frangible, par exemple en formant une boucle connectée de chacun de ses côtés, avec le reste de la partie conductrice située à l'extérieur de ladite zone.

Par feuille de grande flexibilité, on entend une feuille repliable sur elle-même en tous points avec un très petit rayon de courbure, comme une feuille de papier de faible grammage (80g ou inférieur).

A noter que le terme mesure d'impédance est utilisé dans l'invention de façon générale et peut en fait être ramené à une mesure de la résistance du circuit, avec une pile d'alimentation en courant continu.

Le principe consistant à mesurer la résistance de l'ensemble d'un circuit qui, petit à petit, voit son impédance augmenter, par palier correspondant à la valeur calibrée de chaque résistance mise en parallèle, au fur et à mesure que les médicaments sont extraits, et ce, quelque soit la pilule extraite au début ou à la fin de la chaîne, permet ainsi une mesure fiable et simple grâce à un logiciel intégré dans le circuit, logiciel dont la conception est à la portée de l'homme du métier, au vu du problème posé.

Du fait de l'utilisation d'un circuit imprimé métallique, la fiabilité de la mesure est renforcée. En effet il est ainsi possible d'utiliser des résistances électroniques dont la valeur est précise, calibrée, invariable d'une résistance à l'autre et stable dans le temps ce qui est essentiel pour bien identifier le nombre de pilules prises en cas d'expulsion de plusieurs pilules de façon quasi simultanée.
C'est par exemple le cas du patient qui a oublié de prendre son médicament et qui pour ne pas en informer son médecin, va vider ses blisters peu avant la consultation de suivi.

La disposition des portions conductrices au bord des zones frangibles, généralement en aluminium et potentiellement conducteur, entraînent par ailleurs, en combinaison avec le reste des caractéristiques, une impossibilité ou quasi impossibilité de court-circuit.

Dans des modes de réalisation avantageux de l'invention, on a de plus recours à l'une et/ou à l'autre des dispositions suivantes :
- au moins une portion conductrice formant une languette en U à cheval sur le bord de la portion frangible, les points de rupture sont situés entièrement à distance du bord de ladite portion frangible, à l'extérieur de celle-ci ;
- les points de rupture sont situés dans une zone et au niveau de deux orifices situés de part et d'autre des branches du U, au moins un orifice supplémentaire étant situé entre les branches du U ;
- les orifices sont formés par des trous circulaires de diamètre compris entre 0,5 mm et 2 mm ;
- la portion frangible comprend une pastille prédécoupée, délimitée par deux portions évidées symétriques en forme de banane finissant sur des orifices situés à distance d de la périphérie ;
- les extrémités des évidements en forme de bananes sont prolongées par le perçage de plusieurs petits trous tangentiels formant une pyramide ou arc de cercle dont le sommet est formé par un orifice situé entre les deux branches du U ;
- la distance des points de rupture par rapport au bord est comprise entre 1 mm et 3 mm ;
- chaque portion conductrice forme une languette en U traversant la portion frangible correspondante de part en part, les quatre points de rupture situés au bord de ladite portion frangible étant formés pour deux d'entre eux au niveau de la barre du U et pour les deux autres au niveau des sommets des branches du U ;
- une couche intermédiaire comprend un film isolant et un adhésif du film isolant, est intercalée entre le film craquable du blister et la plaquette sur et autour du bord périphérique de la portion frangible ;
- le support est prévu pour au moins une rangée de pilules, chaque alvéole étant pelable par arrachage à partir d'un coin et comprend des parties évidées polygonales en vis à vis dudit coin, au moins un sommet du polygone étant terminé par une portion de ligne de découpe entre deux portions frangibles pour alvéoles adjacentes comportant une partie terminale formant un angle d'échappement agencé pour amorcer et guider le déchirement du blister ;
- le support est prévu pour deux rangées de pilules dont chaque alvéole est pelable par arrachage à partir d'un angle (ou coin) et comprend des évidements centraux en forme de losange disposés au centre de chaque jeux de quatre portions frangibles pour alvéoles adjacentes pour accès à ces angles, les sommets latéraux de chacun des losanges étant respectivement terminés par une portion de ligne de découpe entre deux portions frangibles pour alvéoles adjacentes comportant une partie terminale formant un angle d'échappement agencé pour amorcer et guider le déchirement du blister ;
- chaque portion conductrice comprend une branche entièrement externe à la portion frangible, de sorte que la lecture en transparence du blister est facilitée ;
- chaque portion frangible est formée par une languette centrale découpée sur les cotés par des zones d'évidemment agencées pour corriger éventuellement un décalage dans le collage du blister sur la plaquette ;
- chaque portion frangible comporte une languette centrale découpée sur les cotés par de simple trait de coupes le long des quels sont régulièrement répartis des trous agencés pour faciliter l'éjection de la pilule ;
- les moyens de fixation sont formés d'une couche de colle protégée par un film plastique pelable avant fixation du blister sur la plaquette ;
- la plaquette est en Polyéthylène Naphtalate (PEN), ou dans d'autres modes de réalisation, en polyimide ou en polyester ;
- les portions conductrices propres à être au droit ou sensiblement au droit des pilules du blister et à être rompues lorsque celles-ci sont éjectées manuellement par un utilisateur, sont formées par des pattes à deux jambes détachables reliées entre elles par des fils de jonction via une portion centrale, lesdits fils de jonction étant fragilisés par des perforations réalisées à leur niveau dans la plaquette ;
- le dispositif comporte des moyens de calculs agencés pour retester régulièrement l'impédance ;
- les moyens pour retester périodiquement et/ou régulièrement l'impédance globale comprennent des moyens de comparaison d'au moins une première tension moyenne Tᵢ sur n mesures, avec une tension moyenne précédente Tᵢ₋₁ sur n mesures ;
- n est compris entre 5 et 10, et/ou au moins deux couples de tensions moyennes consécutifs sont vérifiés pour valider une sortie de pilule.

L'invention propose également un procédé de mise oeuvre du dispositif décrit ci-avant.

L'invention propose également un dispositif de mesure de l'observance comprenant une plaquette équipée d'un circuit électronique de détection et d'enregistrement du moment d'extraction des pilules contenues dans des alvéoles appartenant à un blister et des moyens de fixation du blister à la plaquette par collage, la plaquette étant formée d'un support comportant la partie conductrice dudit circuit pour former les interrupteurs propres à être ouverts lorsque les pilules sont extraites manuellement par un utilisateur, **caractérisé en ce que** le support est prévu pour au moins une rangée de pilules, chaque alvéole étant pelable par arrachage à partir d'un coin et comprend des parties évidées polygonales en vis à vis dudit coin, au moins un sommet du polygone étant terminé par une portion de ligne de découpe en vis à vis de la liaison entre deux alvéoles adjacentes comportant une partie terminale formant un angle d'échappement agencé pour amorcer et guider le déchirement du blister.

Avantageusement le support est prévu pour deux rangées de pilules dont chaque alvéole est pelable par arrachage à partir d'un angle et comprend des évidements centraux en forme de losange disposés au centre de chaque jeux de quatre alvéoles adjacentes pour accès à ces angles, les sommets latéraux de chacun des losanges étant respectivement terminés par une portion de ligne de découpe entre deux alvéoles adjacentes comportant une partie terminale formant un angle d'échappement agencé pour amorcer et guider le déchirement du blister.

L'invention sera mieux comprise à la lecture de la description qui suit de modes de réalisation donnés ci-après à titre d'exemples non limitatifs.

La description se réfère aux dessins qui l'accompagnent dans lesquels :
La figure 1 montre schématiquement les étapes de mise en oeuvre d'un dispositif selon un mode de réalisation de l'invention.
Les figures 2 et 3 sont des vues en plan de deux modes de réalisation de plaquettes appartenant à un dispositif selon l'invention.
Les figures 2A et 3A montrent schématiquement, en vue de dessus et agrandie, deux portions frangibles avec leurs portions conductrices associées, correspondant respectivement aux figures 2 et 3.
La figure 4 montre une portion conductrice à jambes détachables, (au niveau des doubles zones de rupture) selon un mode de réalisation de l'invention.
La figure 5 est une vue en plan d'un autre mode de réalisation d'une plaquette selon l'invention.
Les figures 6A, 6B et 6C sont respectivement une vue en plan et deux vues schématiques latérales en coupe (avant et lors de l'éjection d'une pilule) d'une portion frangible selon un autre mode de réalisation de l'invention.
La figure 7 est un schéma bloc montrant un mode de réalisation du test anti-faux positif, mis en oeuvre selon le mode de réalisation de l'invention plus particulièrement décrit.
La figure 8 montre en vue de dessus, une portion frangible avec deux portions conductrices selon un mode de réalisation de l'invention.
La figure 9 est une vue schématique de la figure 8 après rupture.
La figure 10 est une vue schématique en coupe de la figure 8 selon X-X.
La figure 11 est une vue de dessus partielle d'un mode de réalisation d'une plaque selon l'invention.

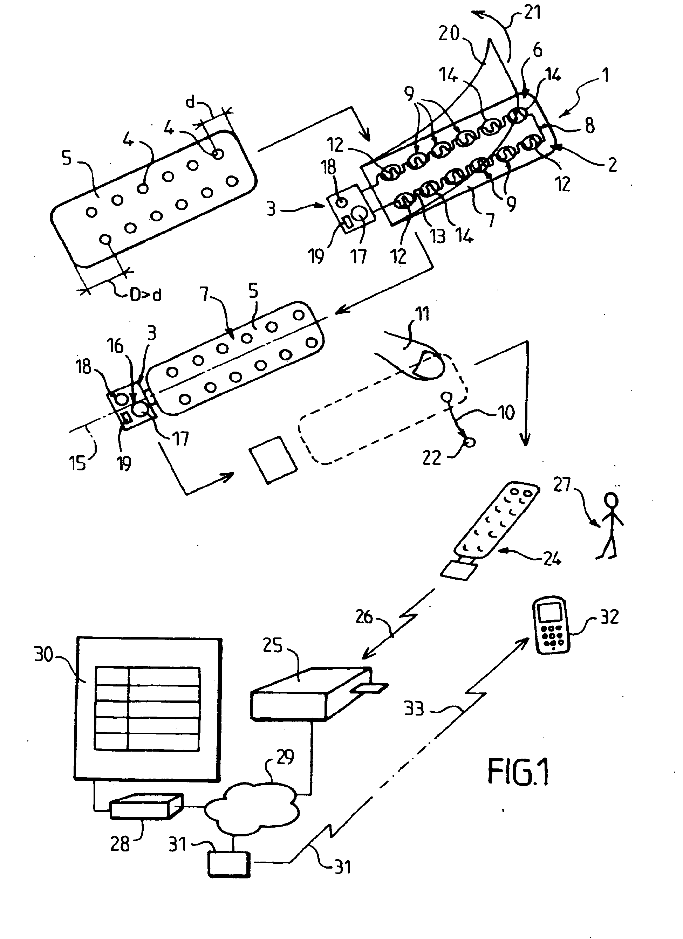

La figure 1 montre un dispositif 1 de mesure de l'observance comprenant une plaquette 2 équipée d'un circuit 3 électronique de détection et d'enregistrement du moment d'éjection des pilules 4 appartenant à un blister 5.

Le blister 5 est formé de façon connue d'une feuille souple comprenant par exemple deux rangées parallèles de six pilules 4.

Le dispositif comprend des moyens 6 (colle enduisant la face supérieure de la plaquette) de fixation du blister à la plaquette. La plaquette est formée d'un support souple 7 sur lequel est imprimée la partie conducteur 8 du circuit électronique pour former des interrupteurs 9 propres à être ouverts lorsque les pilules sont éjectées manuellement (voir flèche 10) par un utilisateur dont le pouce 11 est représenté sur la figure 1.

Plus précisément, le circuit électronique 3 comporte des portions conductrices 12 identiques réparties régulièrement le long du chemin conducteur 13.

Le support est souple, flexible, transparent, sensiblement rectangulaire, et comprend des portions frangibles 14, par exemple oblongues, en bordure desquelles sont imprimées les portions conductrices 12, réparties régulièrement pour être sensiblement au droit d'une pilule 4 correspondante lorsque le blister 5 est fixé sur la plaquette, de sorte que la portion frangible de la plaquette et la portion conductrice correspondantes sont rompues simultanément lorsqu'une pilule est éjectée.

La partie conductrice est constitué d'un aplat cuivré étamé adhérent à son support souple de 0,2 à 0,5 mm de largeur.

Le support transparent est plus précisément et quant à lui, une feuille mince (< 0,1 mm, par exemple 0,05 mm) totalement souple et indéchirable, en Polyéthylène Naphtalate (PEN), ou encore formé d'un polyimide ou de polyester ou tout autre support souple en feuille de matière plastique ou autre, formant un complexe compatible avec un contact avec un médicament.

Le circuit 3 comporte des moyens 16 de mesures de l'impédance globale de la partie conducteur pour en déduire le moment de l'extraction des comprimés ou pilules.

Ces moyens 16 comportent une pile d'alimentation électrique 17, une puce 18 électronique de mesure programmée en conséquence et un ensemble 19 de constituants électroniques : mémoire, quartz, résistance et antenne émettrice à radiofréquence ( puce RFId) notamment.

Les moyens 16 sont constitués d'éléments connus, raccordés et programmés de façon à la portée de l'homme du métier.

Les moyens 6 de fixation sont formées d'une couche de colle protégée par un film plastique 20 pelable (flèche 21) avant fixation du blister 5 sur la plaquette.

Avantageusement une couche d'isolant électrique (non représentée) et/ou de résine ou de vernis isolant, est ajoutée au préalable entre la couche de colle et la plaquette.

Ainsi, quand le patient prend une pilule 22, la date et l'heure exacte sont enregistrées par la puce 18 dans la mémoire 19. Un appareil lecteur à radiofréquence 25, utilisant les ondes radio électromagnétiques 26 émises par la puce RFId, est par exemple prévu chez le patient 27, et permet une retransmission des informations au serveur distant 28 par exemple via internet 29 pour former un fichier 30 regroupant les données d'observance du patient 27, données qui vont être par exemple retransmises à l'ordinateur 31 d'un médecin via internet.

Avantageusement des instructions de suivi au patient 27 par exemple par SMS sur son téléphone portable 32 sont alors retransmises (flèche 33) par le médecin au patient.

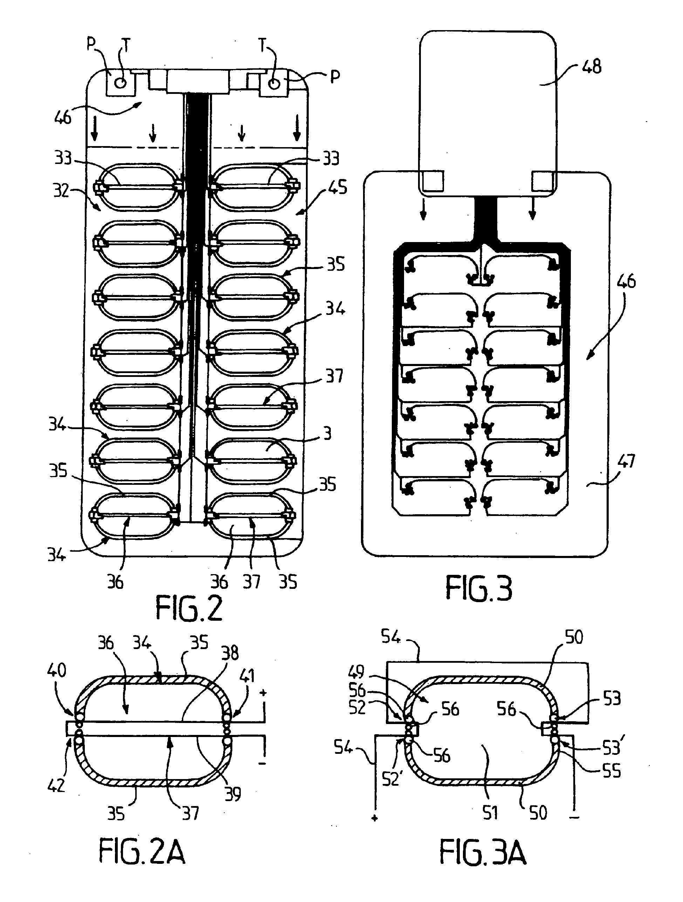

On a représenté sur les figures 2, 2A et 3, 3A deux autres modes de réalisation d'éléments d'un dispositif selon l'invention, de façon non limitative.

Plus précisément, la figure 2 montre une plaquette 32 comprenant le chemin conducteur 33 d'un circuit électrique selon un mode de réalisation de l'invention.

Ici les portions frangibles oblongues 34 comprennent deux zones de fraisage ou plus généralement de découpe 35 de part et d'autre, laissant une grande languette ovale 36 au centre, correspondant à l'orifice de sortie de la pilule.

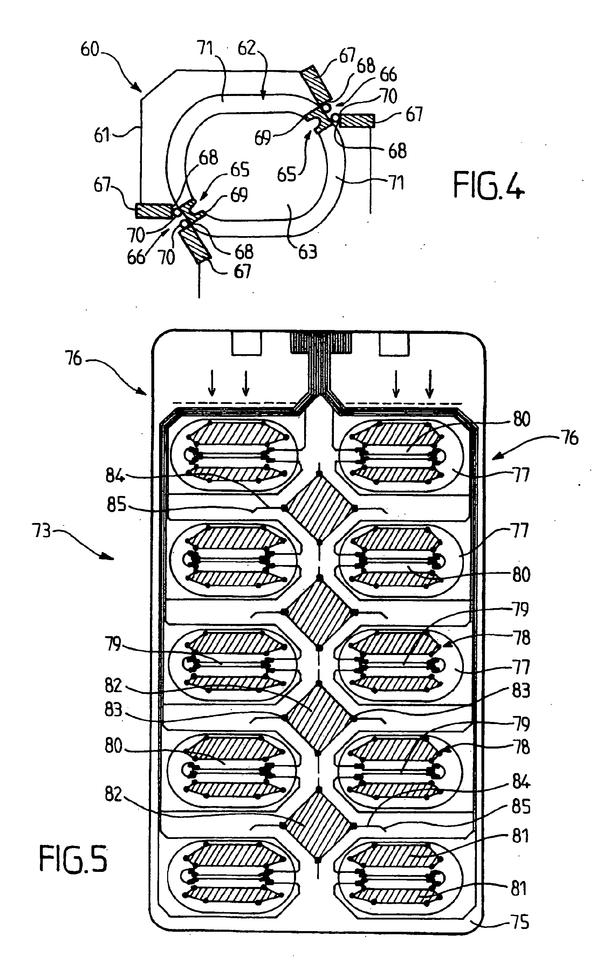

La portion conductrice en parallèle 37 est montrée agrandie sur la figure 4A. Elle est formée par exemple par une trace de cuivre de 0,2 mm de largueur.

Dans ce mode de réalisation elle présente une forme en U dont les deux branches 38 et 39 forment une boucle qui traverse la languette 36 de part en part, la barre du U 40 étant à l'extérieur de l'évidemment, en présentant à chaque extrémité et à cheval sur le bord dudit évidement de la portion frangible 34, des zones de rupture 41, 42 chacune obtenue par quatre petits trous ronds 43, 44, alignés, à savoir deux trous externes de diamètre 1,2 mm et deux plus petits trous internes de diamètre 0,8 mm.

Les zones de fraisage 35 prennent en compte et corrigent le décalage possible dans le positionnement de l'étiquette par rapport au blister.

Une telle disposition en U présente l'avantage d'économiser la place sur les côtés 45 de la plaquette en feuille 32, présentant sa partie connectique 46 avec le boîtier (non représenté) qui contient l'électronique.

Il y a ainsi quatre interrupteurs (deux fois deux possibilités de rupture , i.e. deux en 41 et deux en 42), ce qui augmente d'autant les possibilités de cassures, et ce ici grâce aux trous différents.

La figure 3 montre schématiquement un autre mode de réalisation du chemin conducteur 46 d'un dispositif selon l'invention.

Seul le chemin conducteur est ici représenté sur la feuille 47 en matière plastique type PEN fixée au boîtier 48.

Les moyens de fixation entre boitier et feuille de plastique sont mécaniques et assurent une grande solidité de jonction entre eux. Ils sont par exemple formés par deux pinoches en excroissance situées sur la partie interne de la moitié du couvercle dudit boitier, qui traversent la feuille 32 ou 47 de part en part par le biais de trous T en vis à vis, percés dans des renforts carré ou petites plaques P situées au bord de ladite feuille, (voir figure 2) pour s'encastrer dans des orifices correspondant de l'autre moitié du couvercle, les deux moitiés étant de plus et par exemple fixées l'une avec l'autre par collage.

La figure 3B montre plus précisément un évidement 49, muni comme pour le mode de réalisation précédent de parties évidées 50 entourant une languette transparente ovale 51.

Toute la zone de la languette 51 n'étant pas traversée par le chemin conducteur elle reste totalement transparente, et on peut ainsi lire plus facilement au travers, ce qui est avantageux pour certains blisters pour lesquels des informations importantes sont imprimées au centre de l'alvéole contenant la pilule.

Ici le chemin présente deux doubles interrupteurs 52, 52' et 53, 53', de part et d'autre de l'évidemment 49, le chemin électrique 54 passant à l'extérieur de l'évidement.

Les points de fragilisation du chemin 54, à cheval sur le bord 55 de l'évidement sont par exemple formés par des petits trous 56, comme décrit précédemment, par exemple deux plus gros trous, séparés par deux plus petits, le chemin 54 passant entre les trous, les deux plus petits étant situés entre les petites deux branches des fils qui forment donc deux boucles en U.

La figure 4 montre un mode de réalisation des parties frangibles 60 et portions conductrices 61 selon l'invention.

Ici, les portions frangibles 60 comportent un évidemment oval 62, une pastille centrale 63 également ovale de plus petite dimension que l'évidemment, reliée au reste de la plaquette 64 par des petites portions en feuille plastique sensiblement rectangulaires 65 qui comportent les zones de jonctions conductrices 66. Les zones de ruptures forment des pattes à deux jambes détachables 67, reliées entre elles par des fils de jonction 68 via une portion centrale 69 plus large, appartenant à la pastille.

La fragilité de ces zones de jonctions conductrices (fil de jonction 68) et détachables est crée, d'un coté (entre les jambes) par des petites perforations 70 et de l'autre coté par des zones 71 évidées en croissant, autour de la pastille, ou encore par quatre perforations de diamètres calculés pour permettre une rupture adaptée, à savoir deux trous de plus fort diamètre sur l'extérieur, et de plus faible diamètre sur l'intérieur des jambes 67.

On a représenté sur la figure 5 une plaquette 73 selon un autre mode de réalisation.

La plaquette 73 comporte un support rectangulaire en feuille souple 75 prévu pour deux rangées de pilules, par exemple deux rangées de cinq pilules.

Pour ce faire elle comprend deux rangées 76 de cinq zones frangibles ovales 77 chacune munie d'une portion conductrice 78 du type décrite en référence à la figure 2A.

Ici la partie conductrice en U 79 est portée par une languette 80 centrale sensiblement rectangulaire comprenant de part et d'autre des évidements 81 occupant au moins le tiers de la surface de l'évidement.

Dans ce mode de réalisation la plaquette comporte de plus des évidements centraux 82 en forme de losange disposés au centre de chaque jeu de quatre zones frangibles pour pilules adjacentes, les sommets latéraux 83 de chacun des losanges étant respectivement terminé par une portion 84 de ligne de découpe entre deux pilules adjacentes comportant une partie terminale 85 formant un angle d'échappement (par exemple de 30°), agencé pour amorcer et guider le déchirement du blister.

Une telle disposition dont l'agencement est ici non limitatif, est lié au type de blister et de médicament qu'il comporte.

Dans l'exemple plus particulièrement décrit ici les losanges correspondent aux zones qui permettent de saisir les coins, non collés, que le patient doit relever, saisir puis tirer pour peler et donc décoller et enlever la partie protectrice bouchant le fond de l'alvéole pour libérer la pilule.

Pour un médicament comportant un emballage sous blister de ce type, comme celui connu sous la marque Pradaxa® il se trouve que les coins sont disposés par quatre et que cela forme un losange.

Mais d'autres dispositions des « coins » à saisir sont possibles.

Quand le patient tire un coin (et quand il a collé auparavant la plaquette à l'étiquette électronique selon l'invention), non seulement, il dégage la pilule, mais il coupe alors forcément la trace de cuivre à proximité menant à l'interrupteur de l'alvéole concernée, à cause de la prédécoupe judicieusement orientée.

Dans ce cas bien sûr, on enregistre une prise sans pour autant que la partie « interrupteur » en U soit ouverte. Le courant est en effet interrompu car la trace menant à l'interrupteur est coupée lorsque le patient tire le coin.

Pour Pradaxa®, la notice est explicite ; le patient doit sortir son médicament en tirant un coin.

Mais malheureusement, les patients ne lisent pas les notices et vont en général tenter d'extraire leurs pilules comme ils pourront.

Beaucoup font comme d'habitude, en pressant comme des forcenés sur le coté du blister présentant des alvéoles.

Avec ce système selon ce mode de réalisation de l'invention il est donc tenu compte de tous les comportements possibles.

Ce type de mode de sortie d'une pilule en tirant un coin étant par ailleurs de plus en plus fréquent pour des raisons différentes à Pradaxa® (il y a de plus en plus de pilules qui ne sont ni des comprimés ni des gélules, mais plutôt des pastilles très friables en général prévues pour être mises sous la langue pour se diluer instantanément).

Dans ce cas, l'extraction d'une pilule (friable) en pressant coté alvéole risque d'abîmer le médicament. Les patients le font une fois et comprennent que cela n'est pas la bonne solution.

Après, ils utilisent un coin comme prescrit par la notice. Dans ce cas les interrupteurs sont supprimés et remplacés par des prédécoupes orientées.

Les évidements 81 de part et d'autre de la languette 80 sont importants. L'objectif ici est de ne pas gêner le craquage du film protecteur obturant les alvéoles. Sans ces évidements occupant une surface importante de la surface de l'alvéole, le patient ne pourrait plus extraire une pilule en craquant le film protecteur si ce dernier est assez résistant.

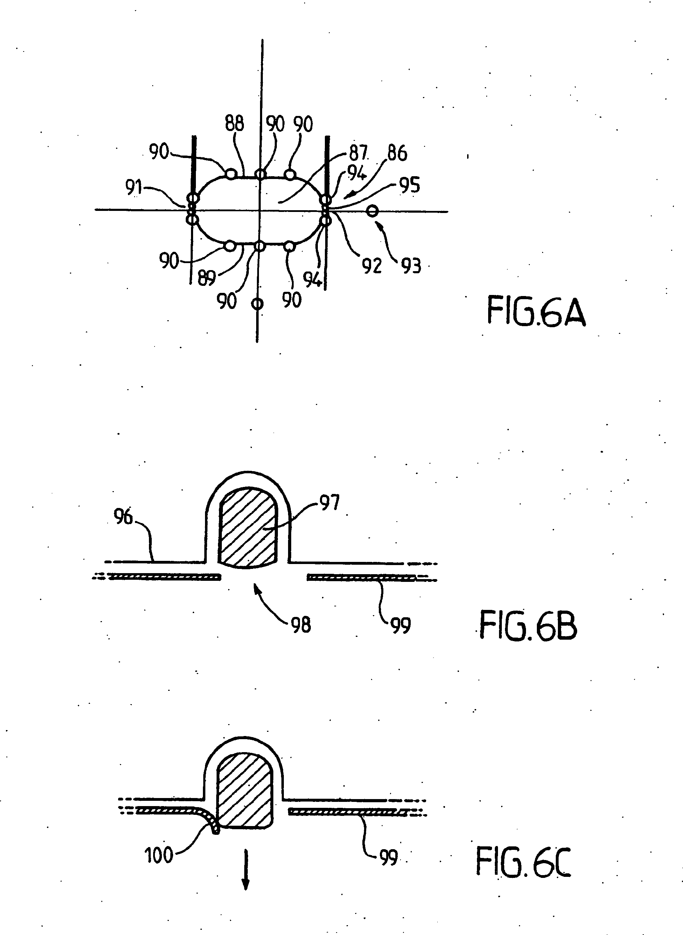

Les figures 6A à 6C montrent un autre mode de réalisation des portions frangibles utilisables avec l'invention. Ici chaque portion frangible 86 est formée par une languette centrale 87 découpée sur les deux cotés opposés 88 et 89, par de simples traits de coupe le long desquels sont régulièrement répartis des trous ronds 90, par exemple deux fois trois trous de 1,2 mm de diamètre, a cheval en partie à l'extérieur de ladite ligne de coupe.

Deux petites portions 91 et 92, de liaison avec le reste de la plaquette 93 sont prévues pour l'impression de la partie conductrice, la fracture de cette portion étant fragilisée par deux trous d'arrêt 94 de part et d'autre de celle-ci, deux petits trous 95 pour permettre les quatre points de rupture étant par ailleurs prévus entre les deux trous 94, comme décrit en référence aux figures 2A et 3A.

Les figures 6B et 6C montrent le fonctionnement de l'éjection d'une pilule.

Dans le cas où (figure 6B) le blister 96 dont l'alvéole contient le comprimé 97 est décalé par rapport à la partie frangible 98 du support 99, un effet volet 100 (cf figure 6C) est observé, qui protège l'environnement immédiat de tout risque de déchirement.

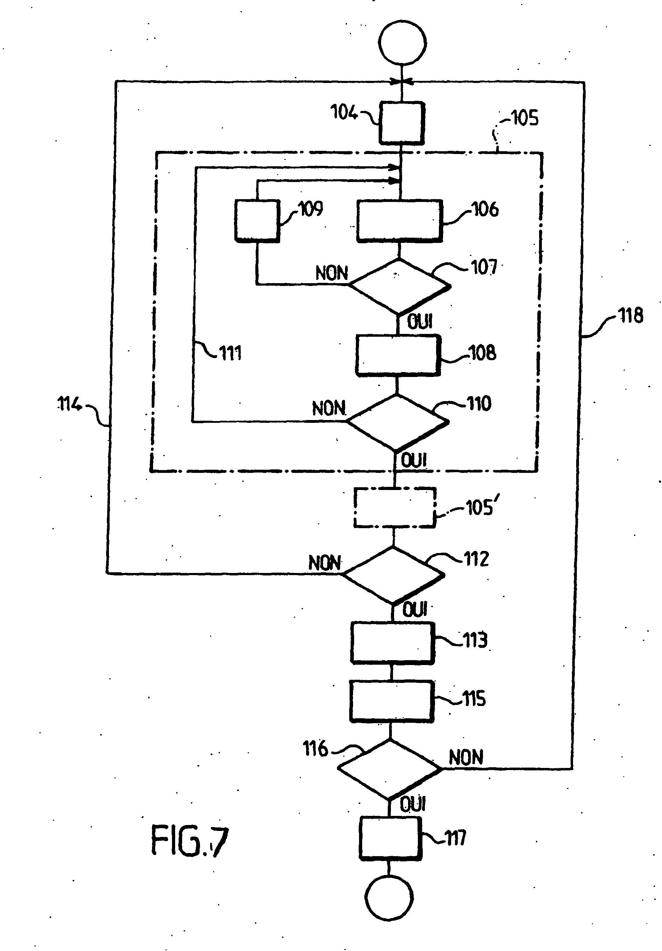

On va maintenant décrire en référence à la figure 7 les moyens de calculs et plus particulièrement de tests selon un mode de réalisation de l'invention permettant d'éviter les faux positifs et/ou faux négatifs de façon extrêmement efficace.

On initialise tout d'abord dans un premier temps en 104 le processus de suivi.

Puis, est réalisé un cycle 105 (trait mixte) de mesures de tension, de la façon suivante. On mesure en 106 de façon périodique, par exemple toutes les secondes, la tension T du circuit du dispositif.

Lorsque le nombre de mesures est inférieur à n (test 107), on stocke la mesure en 109, et on recommence le test.

Si le nombre de mesure devient égal à n, on effectue en 108 la moyenne des tensions T indice i sur les n mesures à partir des mesures stockées en 109, valeur moyenne Tᵢ que l'on stocke alors dans une mémoire tampon du circuit électronique inclus dans le boîtier en plastique.

On effectue alors le test 110 dans lequel on compare les moyennes de T indice i et T indice i-1.

Si cette comparaison montre que cette valeur est inférieure à une valeur seuil déterminée au préalable, on effectue un nouveau cycle de tests identique au cycle 105 en 105', sinon (ligne 111), on revient à la mesure 106 périodique de la tension.

Dans le cas où la deuxième mesure moyennée de tension est toujours différente de la tension indice i moins 1 (test 112) au delà de la valeur seuil, on considère qu'il y a effectivement prise d'une pilule et on stocke (bloc 113) les éléments la concernant dans la mémoire des moyens de calcul. Sinon on réitère le processus en réinitialisant les séries de mesures en 104 (ligne 114).

Lorsque cette mesure de prise est stockée, elle est ensuite éventuellement soumise à différents calculs (blocs 115) qui vont pouvoir être par exemple copiés par le biais d'une lecture de la puce RFID sur un dispositif externe non représenté.

A la fin de la mesure et/ou de l'utilisation de la totalité de la plaquette (test 116) on transmet (étape 117) les informations à un serveur distant et/ou la plaquette au médecin pour analyse.

Sinon on réinitialise (ligne 118) le fonctionnement.

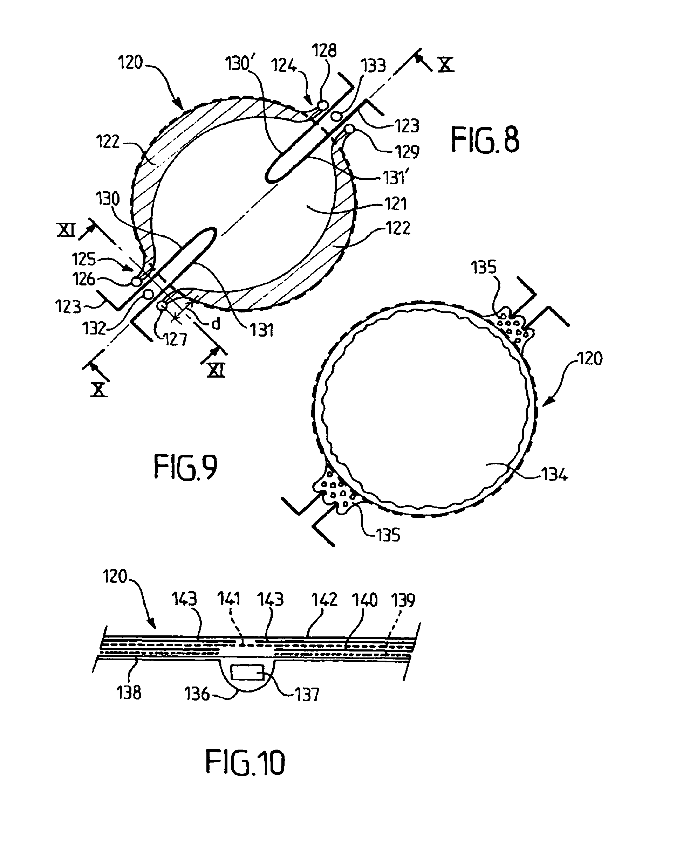

La figure 8 montre les limites ou périphérie 120 d'une alvéole ici circulaire, ce qui correspond plus ou moins à la zone de déchirure du film en aluminium (ou équivalent) lors de la sortie d'une pilule.

Les limites 120 correspondent sensiblement à la position et aux dimensions des alvéoles du blister pour laquelle l'étiquette est destinée. Le collage de l'étiquette sur son blister peut accepter une certaine imprécision de positionnement qui peut dépasser les 2/3 millimètres lorsque les alvéoles sont éloignées les unes des autres.

La zone de déchirure du film aluminium est figurée en dedans par un trait plein ondulant dans la figure 9.

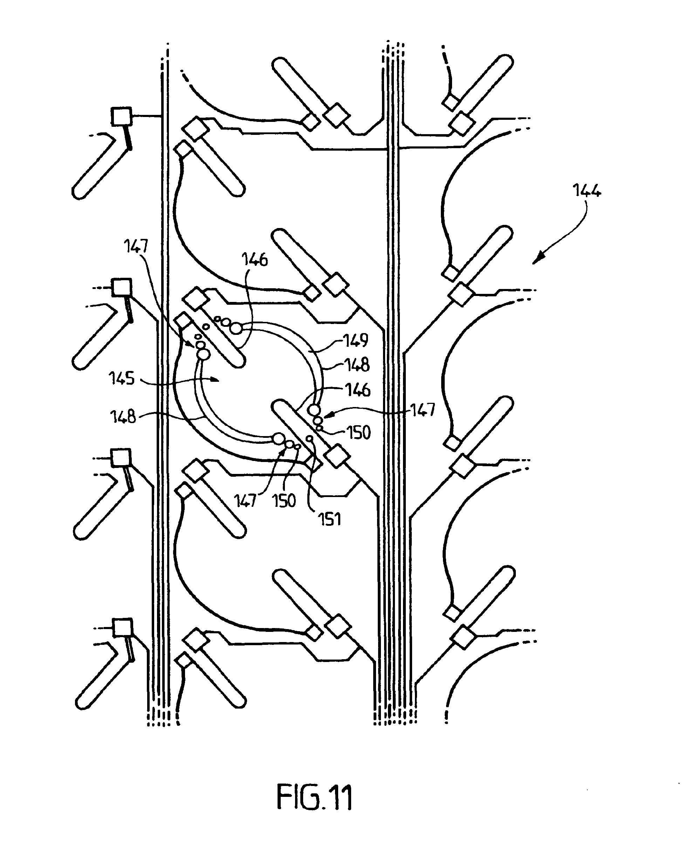

La pastille 121 prédécoupée est délimitée par deux portions évidées 122 symétriques en forme de croissant ou de banane et finissant sur les trous 128, 129, 126 et 127 à la distance d de la périphérie (à noter que les extrémités des évidements en forme de bananes peuvent être aussi prolongées par le perçage de plusieurs petits trous tangentiels comme sur la fig 11), les trous de diamètres décroissants par exemple, formant une pyramide ou arc de cercle dont le sommet est formé par l'orifice 151, par exemple plus petit, situé entre les deux branches du U de la façon la plus éloignée par rapport au bord 120.

Le nombre d'interrupteurs est de deux sur la figure 8 mais ce nombre dépend de la forme, la taille et la disposition des alvéoles.

Ce nombre peut par exemple varier de un à quatre. La pastille 121 est rattachée au reste de l'étiquette par la partie de matière restante entre les trous 128 et 133, 133 et 129, 126 et 132 et enfin 132 et 127. Chacun des trous ou orifices 128, 129, 130, 131, 132, 133, sont des perforations de l'étiquette par exemple de 0,8 mm de diamètre (seul le film constituant -sur lequel adhère la trace 123- de l'étiquette et non la couche d'adhésif est perforé).

La distance entre deux trous est par exemple de l'ordre de 3 à 4 dixièmes de millimètres, distance suffisante pour laisser passer la trace conductrice d'environ 2 dixièmes de millimètres et deux petits espaces entre la trace elle-même et chaque trou, ces petits espaces sans trace permettant de minimiser le risque de court-circuit entre la trace, après sa rupture, et le film (ou un déchet de celui-ci) en aluminium.

Les deux portions conductrices 123 (chaque portion conductrice correspondant à un interrupteur, le nombre de portions conductrices peut donc aussi varier de un à quatre) forment deux languettes en U et traversent la limite figurée en pointillée.

Les lieux de rupture des traces 123 (la rupture étant secondaire à la sortie d'une pilule d'un blister) sont situés entre chaque couple de trous : 128 et 133, 133 et 129, 126 et 132 et enfin 132 et 127.

La sortie d'une pilule doit entrainer la rupture d'au moins une branche d'une trace 123 pour pouvoir être enregistrée mais la rupture au niveau de toutes les paires de trous n'est pas systématique et dépend de la façon, possiblement et toujours très variable, dont le patient s'y prend pour extraire sa pilule.

Comme indiqué selon ce mode de réalisation de l'invention plus particulièrement décrit les lieux possible de rupture des traces 123 sont situés à une distance d du bord périphérique 120, par exemple 1,5 mm.

La fig 9 montre les traces 123 après rupture (la pastille centrale a ici été enlevée mais pourrait rester accrochée d'un côté de la pastille - cas où une seule trace 123 a été rompue-). Les extrémités restantes de la trace 123 (l'extrémité non positionnée sur la pastille centrale), toujours reliées électriquement à la partie électronique de mesure, est un morceau de cuivre à vif et non protégé qu'il est important de ne pas mettre en contact avec le film aluminium du blister.

Le décalage d sus-évoqué vers l'extérieur permet d'écarter l'extrémité de la trace 123 du film rompu en aluminium ce qui permet de minimiser le risque de court-circuit.
La figure 9 montre donc ce qui ce passe après sortie d'un comprimé qui a entrainé le détachement de la pastille centrale.

Un film d'aluminium 134 ou équivalent du blister est rompu de façon irrégulière, la surface 135 qui correspond à une partie évidée de plastique jouant à cet endroit un rôle d'isolant vis à vis du fil qui, étant rompu à distance de la pellicule d'aluminium, ne risque plus de générer des courts-circuits entre le fil ou trace de cuivre et d'aluminium conducteur du blister. Notons qu'au niveau de cette partie 135, le film en aluminium n'a pas pu être arraché car il adhère fortement au blister.

Mais, il reste recouvert au niveau de la surface 135 par la couche d'adhésif car la couche d'adhésif adhère plus au film d'aluminium qu'à la pastille de l'étiquette. L'arrachement de la pastille ne provoque ainsi pas l'enlèvement de la masse adhésive, ce qui contribue à minimiser le risque de court-circuit.

Pour les contextes d'utilisation où la fiabilité de l'étiquette doit être encore accrue, il est possible de rajouter un film isolant supplémentaire : celui-ci correspondra à l'étiquette dont il fera partie mais sera évidé au droit des bananes et de la pastille sauf au niveau de la surface 135.

On a représenté sur la figure 10 en coupe XX la région de la portion frangible 120.

Le blister comprend l'alvéole 136 qui contient la pilule 137.

L'alvéole est fermée par le film d'aluminium ou équivalent 138. Un adhésif 139 permet de fixer un film isolant 140 qui ne recouvre pas l'alvéole mais est présent sur le reste de l'étiquette notamment au niveau de la surface 135 figurée sur la fig 9, puis une couche d'adhésif 141 permet de fixer la feuille de plastique 142, les portions conductrices 143 étant représentées en discontinu. La présence du film isolant 140 n'est pas constante.

La figure 11 montre une portion de feuille 144 avec la pastille 145, les portions conductrices en U 146 et les points de rupture agencés entre les trous 150, 151 et 150 à distance du bord 148 de la position théorique du bord d'une alvéole.

Ici les trous sont au nombre de 7, à savoir deux trous supplémentaires à l'extrémité des bananes évidés 149 comme décrits ci-avant, quatre trous 150 en chapelet de part et d'autre des branches du U et un trou central 151, à distance d du bord 148.

Dans ce mode de réalisation et pour décentrer les points de rupture, ou interrupteurs, on a donc percé des trous intermédiaires tout en emmanchonnant la trace de cuivre du fait de :
- ce décentrement tout d'abord qui crée une sorte de cavité virtuelle 135 à la périphérie de l'alvéole, diminuant les risques d'avoir un morceau résiduel de film en aluminium resté solidaire du blister qui revienne en contact avec le fil ou trace ;
- une épaisseur d'adhésif isolant, ensuite du type aboutissant au fait que, après arrachement de la pastille, l'adhésif reste sur le film en aluminium ;
- éventuellement ce film isolant supplémentaire 140, ledit film présentant des orifices au droit de passage des pilules, placé entre les interrupteurs excentrés et le circuit imprimé.

On va maintenant décrire plus précisément un exemple de réalisation du fonctionnement de l'élimination de faux positifs et/ou négatifs.

Pour se faire on a par exemple mis en mémoire trente seuils correspondant aux trente prises à effectuer sur une plaquette.

Trente prises est en général un nombre maximum de pilules pour les blisters du commerce.

Plus généralement les blisters comportent quatorze ou quinze pilules.

Pour se faire on mesure la tension aux bornes du circuit électronique, tension qui augmente au fur et à mesure des prises, car chaque fois qu'il y a une prise un interrupteur s'ouvre et le courant ne passe plus directement mais à travers une résistance supplémentaire.

En d'autres termes, le circuit augmente sa résistance (également qualifiée d'impédance) au fur et à mesure des prises.

La problématique que se propose de résoudre cet algorithme plus particulier est en fait d'éviter encore plus les faux positifs.

En effet, il a pu être observé qu'il y avait de temps en temps une mesure faussée dont la raison exacte était difficile à déterminer.

Celle-ci peut venir de court-circuits, le circuit étant peu protégé, par exemple juste par une couche de colle, et peut donc venir au contact de la partie aluminium du blister quand les interrupteurs sont rompus.

Par ailleurs, dans le cas d'une utilisation de puce RFID, le système peut détecter de fausses mises en communication et tirer également sur la consommation de la pile qui est incluse dans le dispositif électronique.

Cette pile étant une pile de faible puissance, elle peut alors éventuellement transmettre une tension insuffisante dans le circuit et simuler une prise ou une absence de prise alors que cette dernière a ou n'a pas eu lieu.

Quoiqu'il en soit cette identification de problèmes rencontrés dans le cadre d'une utilisation de blisters du commerce, par des utilisateurs normaux avec ce type de dispositif, est nouvelle.

L'invention le gère par des moyens tels que précisés ci-avant pris en combinaison, qu'elle améliore encore dans le mode de réalisation plus particulièrement décrit, par des moyens de calcul algorithmiques mettant en oeuvre des tests successifs, le contrôle étant réalisé au moins une deuxième fois, éventuellement une troisième fois, etc.

On ne conclura à une prise que lorsque deux mesures successives de moyennes de T, c'est à dire en l'espèce dans l'exemple mentionné deux fois huit mesures moyennées, permettent de conclure consécutivement à la dite prise.

Avec le dispositif selon ce mode de réalisation de l'invention, on a ainsi pu constater un seul faux positif sur plus de six cent prises, la fiabilité pouvant encore être améliorée en augmentant le nombre de cycles 105 de contrôle.

Un autre avantage inattendu provient du fait que, si une connexion ou plusieurs se referment alors qu'il n'y a pas eu de nouvelles prises, par exemple du fait de la manipulation de la plaquette, cela ne crée pas d'erreurs puisque les prises sont stockées en mémoire.

On va maintenant décrire le fonctionnement du dispositif et du procédé selon le mode de réalisation de l'invention plus particulièrement décrit ici en référence à la figure 1.

L'utilisateur (patient) à qui un médicament correspondant au blister 5 a été prescrit, se voit fournir, soit par son médecin, soit par le pharmacien, soit directement par le fabricant du blister, un dispositif 1 selon l'invention.

Celui-ci, fabriqué en grande série, a un coût faible, compte tenu notamment de la simplicité de la puce 17 dite RFID (initiales anglo saxonnes de Radio-Frequency Identification) qui comporte les moyens de calcul, de stockage et de transmission des informations d'enregistrement.

L'utilisateur détache la pellicule 20 sur le dispositif qu'il colle ensuite sur le blister 5, sans se préoccuper du côté du blister plus long au moins long, compte tenu des surfaces frangibles oblongues 9.

Le blister est collé, pilules vers le dessus, la face en feuille d'aluminium étant du côté du dispositif.

Grâce au fait que ce dernier est transparent, les indications figurant au dos du blister sont lisibles au travers de la plaquette 2.

L'utilisateur, au fur et à mesure de son traitement extrait ensuite les pilules 22 du Kit médicamenteux en pressant manuellement sur le blister ou en arrachant un de ses coins dans le mode de réalisation de la figure 5.

En appuyant sur le blister ou en arrachant l'un des cotés, la pilule échappe le film d'aluminium et il y a en même temps rupture de l'une et/ou l'autre des parties de jonction.

L'impédance du circuit est alors augmentée ce qui active le compteur et les enregistrements des prises médicamenteuses.

A la fin des prises et/ou en temps réel les données enregistrées par le blister/dispositif sont transmises au médecin.

Sachant qu'il a été monitoré de façon infalsifiable dans ses prises de médicaments, l'utilisateur est invité à une conduite plus respectueuse de la prescription, ce qui permet une plus grande efficacité du médicament, chez l'utilisateur déambulatoire.

Comme il va de soi et comme il résulte également de ce qui précède, la présente invention n'est pas limitée aux modes de réalisation plus particulièrement décrits. Elle en embrasse au contraire toutes les variantes et notamment celles où le boîtier qui contient les moyens de calcul est amovible et réutilisable, où les pilules sont des comprimés ou des gélules, ou plus généralement des unités thérapeutiques (ampoules... )ou éléments (seringues... ) conditionnés en blister, ou encore celles qui comportent des moyens de guidage du collage du blister sur la plaquette pour un utilisateur. Dans ce cas, et par exemple, le dispositif comporte de plus, une pellicule double arrachable constituée en papier kraft légèrement plastifié ou en feuille de matière plastique apposée sur la face supérieure du circuit, à laquelle il est collé par l'intermédiaire d'une mince pellicule de colle.

La pellicule double arrachable comporte une extrémité de traction par un utilisateur, qui va lui permettre de peler cette pellicule de façon à libérer la face supérieure d'accès aux interrupteurs.

Plus précisément, et par exemple la pellicule ou film pelable en forme de bande repliée sur elle-même de même largueur que la plaquette comporte une première moitié collée sur la face et une deuxième moitié munie de l'extrémité décalée.

Un support amovible en carton comportant une ouverture d'insertion du blister pour collage sur la plaquette munie de la partie conductrice du circuit, agencée pour bloquer le blister en translation par ses bords, l'orifice venant en butée sur les bords périphériques du blister est par exemple prévu.

Le support en carton comporte, de plus, des moyens de guidage et de maintien de l'extrémité de la moitié de la pellicule double arrachable, agencés pour permettre le dégagement de la face de la première moitié et libérer la couche de colle pour autoriser le collage du blister sur le support par simple pression.

Une fois le blister collé l'ensemble blister, plaquette support transparente et boîtier contenant les moyens de calculs peut alors être extrait.

## Revendications

1. Dispositif (1) de mesure de l'observance comprenant une plaquette (2, 32, 47, 73) équipée d'un circuit (3, 33) électronique de détection et d'enregistrement du moment d'extraction des pilules (4, 22, 97) appartenant à un blister (5) et des moyens (6) de fixation du blister à la plaquette, la plaquette étant formée d'un support (7, 75) comportant la partie conducteur (8, 33, 46) dudit circuit pour former des interrupteurs (9) propres à être ouverts lorsque les pilules sont extraites manuellement par un utilisateur, la partie conducteur comprenant un fil imprimé de métal conducteur, le support (7, 75) étant formé d'une feuille de plastique mince, souple, pliable, d'une grande flexibilité, , la plaquette comprenant des portions frangibles (14, 34, 60, 77, 86) découpées sur les cotés et réparties régulièrement pour correspondre à une pilule correspondante, le circuit comportant des moyens (16) de mesure de l'impédance globale de la partie conducteur pour en déduire le moment de l'extraction d'une pilule et le fil comportant des portions conductrices identiques réparties régulièrement en parallèle le long d'un chemin conducteur, le chemin conducteur étant à chaque extraction agencé pour présenter une plus grande résistance que la portion en parallèle,
**caractérisé en ce que** la feuille plastique est transparente, **en ce que** au moins quatre points de rupture (41, 42, 52, 52', 53, 53', 68) de ces portions conductrices sont ménagés par portion frangible et pilule correspondante, au bord desdites portions frangibles, de sorte que la portion frangible de la plaquette et au moins un point de rupture de la portion conductrice correspondante sont rompues simultanément lorsqu'une pilule est extraite
et **en ce que** il comporte des moyens de calculs agencés pour retester régulièrement l'impédance, lesdits moyens comprenant des moyens de comparaison d'au moins une première tension moyenne Tᵢ sur n mesures, avec une tension moyenne précédente Tᵢ₋₁ sur n mesures.

2. Dispositif selon la revendication 1, **caractérisé en ce que** au moins une portion conductrice formant une languette en U à cheval sur le bord de la portion frangible, lesdits points de rupture sont situés entièrement à distance du bord de ladite portion frangible, à l'extérieur de celle-ci.

3. Dispositif selon la revendication 2, **caractérisé en ce que** la distance des points de rupture par rapport au bord est comprise entre 1 mm et 3 mm.

4. Dispositif selon l'une quelconque des revendications précédentes, **caractérisé en ce que** chaque portion conductrice (37) forme une languette en U traversant la portion frangible correspondante de part en part, les quatre points de rupture (41, 42) situés au bord de ladite portion frangible étant formés pour deux d'entre eux au niveau de la barre du U et pour les deux autres au niveau des sommets des branches du U.

5. Dispositif selon l'une quelconque des revendications 2 à 4, **caractérisé en ce que** les points de rupture sont situés dans une zone et au niveau de deux orifices situés de part et d'autre des branches du U, au moins un orifice supplémentaire étant situé entre les branches du U.

6. Dispositif selon la revendication 5, **caractérisé en ce que** les orifices sont formés par des trous circulaires de diamètre compris entre 0,5 mm et 2 mm.

7. Dispositif selon l'une quelconque des revendications précédentes, **caractérisé en ce que** chaque portion frangible comprend une pastille (121) prédécoupée, délimitée par deux portions évidées (122) symétriques en forme de banane finissant sur des orifices (128, 129, 126 et 127) situés à distance d de la périphérie.

8. Dispositif selon la revendication 7, **caractérisé en ce que** les extrémités des évidements en forme de bananes sont prolongées par le perçage de plusieurs petits trous tangentiels formant une pyramide ou arc de cercle dont le sommet est formé par un orifice (151), situé entre les deux branches du U.

9. Dispositif selon l'une quelconque des revendications précédentes, **caractérisé en ce que** une couche intermédiaire comprenant un film insolant et un adhésif du film isolant, est intercalée entre le film craquable du blister et la plaquette sur et autour du bord périphérique de la portion frangible.

10. Dispositif selon l'une quelconque des revendications précédentes, **caractérisé en ce que** le support est prévu pour au moins une rangée de pilules, chaque alvéole étant pelable par arrachage à partir d'un coin et comprend des parties évidées polygonales en vis à vis dudit coin, au moins un sommet du polygone étant terminé par une portion de ligne de découpe entre deux portions frangibles pour alvéoles adjacentes comportant une partie terminale formant un angle d'échappement agencé pour amorcer et guider le déchirement du blister.

11. Dispositif selon la revendication 10, **caractérisé en ce que** le support (75) est prévu pour deux rangées de pilules dont chaque alvéole est pelable par arrachage à partie d'un coin et comprend des évidements centraux (82) en forme de losange disposés au centre de chaque jeux de quatre portions frangibles pour alvéoles adjacentes pour accès à ces coins, les sommets latéraux (83) de chacun des losanges étant respectivement terminés par une portion (84) de ligne de découpe entre deux portions frangibles pour alvéoles adjacentes comportant une partie terminale (85) formant un angle d'échappement agencé pour amorcer et guider le déchirement du blister.

12. Dispositif selon l'une quelconque des revendications 1 à 10, **caractérisé en ce que** chaque portion conductrice comprend une branche entièrement externe à la portion frangible, de sorte que la lecture en transparence du blister est facilitée.

13. Dispositif l'une quelconque des revendications précédentes, **caractérisé en ce que** chaque portion frangible (14, 34, 60, 77) comporte une languette centrale (36, 51, 63, 80) découpée sur les cotés par des zones d'évidemment (35, 50, 71, 81) agencées pour corriger éventuellement un décalage dans le collage du blister sur la plaquette.

14. Dispositif selon l'une quelconque des revendications 1 à 12, **caractérisé en ce que** chaque portion frangible (86) est formée par une languette centrale (87) découpée sur les cotés par de simples traits de coupe (88, 89) le long des quels sont régulièrement répartis des trous (90) agencés pour faciliter l'éjection de la pilule.

15. Dispositif selon l'une quelconque des revendications précédentes, **caractérisé en ce que** les moyens (6) de fixation sont formés d'une couche de colle protégée par un film plastique pelable (20) avant fixation du blister sur la plaquette.

16. Dispositif selon l'une quelconque des revendications précédentes, **caractérisé en ce que** la plaquette est en Polyéthylène Naphtalate (PEN), en polyimide ou en polyester.

17. Dispositif selon l'une quelconque des revendications précédentes, **caractérisé en ce que** les portions conductrices propres à être au droit ou sensiblement au droit des pilules du blister et à être rompues lorsque celles-ci sont éjectées manuellement par un utilisateur, sont formées par des pattes à deux jambes détachables (67), reliées entre elles par des fils de jonction (68) via une portion centrale (69), lesdits fils de jonction étant fragilisés par des perforations réalisées à leur niveau dans la plaquette.

18. Dispositif selon l'une quelconque des revendications précédentes, **caractérisé en ce que**
n est compris entre 5 et 10, et/ou au moins deux couples de tensions moyennes consécutifs sont vérifiés pour valider une sortie de pilule.

## Patentansprüche

1. Vorrichtung (1) zum Messen der Einhaltung mit einer Platte (2, 32, 47, 73), die mit einer elektronischen Schaltung (3, 33) zur Erfassung und Aufzeichnung des Zeitpunkts der Herausnahme der Pillen (4, 22, 97) eines Blisters (5) versehen ist, und mit Mitteln (6) zur Befestigung des Blisters an der Platte, wobei die Platte durch einen Träger (7, 75) gebildet ist, der den leitenden Teil (8, 33, 46) der Schaltung aufweist, um Schalter (9) zu bilden, die geeignet sind, geöffnet zu werden, wenn die Pillen manuell von einem Benutzer herausgenommen werden, wobei der leitende Teil einen gedruckten Draht aus einem leitenden Metall aufweist, wobei der Träger (7, 75) durch eine dünne, weiche, faltbare und hochflexible Kunststofffolie gebildet ist, wobei die Platte zerbrechbare Abschnitte (14, 34, 60, 77, 86) aufweist, die an den Seiten ausgeschnitten und regelmäßig verteilt sind, um einer zugeordneten Pille zu entsprechen, wobei die Schaltung Mittel (16) für die Messung der Gesamtimpedanz des leitenden Teils aufweist, um daraus den Zeitpunkt der Herausnahme einer Pille abzuleiten, und wobei der Draht identische leitende Abschnitte aufweist, die regelmäßig entlang einem leitenden Pfad parallel verteilt sind, wobei der leitende Pfad bei jeder Herausnahme angeordnet ist, um einen größeren Widerstand als der parallele Abschnitt aufzuweisen,
**dadurch gekennzeichnet,**
**dass** die Kunststofffolie durchsichtig ist,
mindestens vier Sollbruchstellen (41, 42, 52, 52', 53, 53', 68) dieser leitenden Abschnitte je zerbrechbaren Abschnitt und entsprechende Pille am Rande der zerbrechbaren Abschnitte gebildet sind, so dass der zerbrechbare Abschnitt der Platte und mindestens eine Sollbruchstelle des entsprechenden leitenden Abschnitts bei der Herausnahme einer Pille gleichzeitig gebrochen werden,
und sie Rechenmittel aufweist, die angeordnet sind, um die Impedanz regelmäßig erneut zu prüfen, wobei diese Mittel Mittel aufweisen, um mindestens eine erste über n Messungen gemittelte Spannung Tᵢ mit einer vorherigen, über n Messungen gemittelten Spannung Tᵢ₋₁ zu vergleichen.

2. Vorrichtung nach Anspruch 1,
**dadurch gekennzeichnet,**
**dass** mindestens ein leitender Abschnitt eine U-förmige Zunge bildet, die den Rand des zerbrechbaren Abschnitts übergreift, wobei die Sollbruchstellen vollständig von dem Rand des zerbrechbaren Abschnitts außerhalb dieses Abschnitts beabstandet angeordnet sind.

3. Vorrichtung nach Anspruch 2,
**dadurch gekennzeichnet,**
**dass** der Abstand zwischen den Sollbruchstellen und dem Rand zwischen 1 mm und 3 mm beträgt.

4. Vorrichtung nach einem beliebigen der vorhergehenden Ansprüche,
**dadurch gekennzeichnet,**
**dass** jeder leitende Abschnitt (37) eine U-förmige Zunge bildet, die den entsprechenden zerbrechbaren Abschnitt durchquert, wobei die vier am Rand des zerbrechbaren Abschnitts angeordneten Sollbruchstellen (41, 42) in zwei Fällen an dem U-Steg und in den zwei weiteren Fällen an den Enden der U-Schenkel gebildet sind.

5. Vorrichtung nach einem beliebigen der Ansprüche 2 bis 4,
**dadurch gekennzeichnet,**
**dass** die Sollbruchstellen in einem Bereich von und an zwei Öffnungen angeordnet sind, welche beidseitig der U-Schenkel angeordnet sind, wobei mindestens eine zusätzliche Öffnung zwischen den U-Schenkeln angeordnet ist.

6. Vorrichtung nach Anspruch 5,
**dadurch gekennzeichnet,**
**dass** die Öffnungen durch kreisförmige Löcher mit einem Durchmesser zwischen 0,5 mm und 2 mm gebildet sind.

7. Vorrichtung nach einem beliebigen der vorhergehenden Ansprüche,
**dadurch gekennzeichnet,**
**dass** jeder zerbrechbare Abschnitt einen vorgeschnittenen Patch (121) aufweist, der durch zwei symmetrische, bananenförmige, ausgenommene Abschnitte (122) begrenzt ist, welche an Öffnungen (128, 129, 126 und 127) enden, die um einen Abstand d von dem Umfang beabstandet sind.

8. Vorrichtung nach Anspruch 7,
**dadurch gekennzeichnet,**
**dass** die Enden dieser bananenförmigen Ausnehmungen durch das Bohren mehrerer kleiner tangentialer Löcher verlängert werden, welche eine Pyramide oder einen Kreisbogen bilden, deren Gipfel bzw. Scheitel durch eine Öffnung (151) gebildet ist, die zwischen den beiden U-Schenkeln angeordnet ist.

9. Vorrichtung nach einem beliebigen der vorhergehenden Ansprüche,
**dadurch gekennzeichnet,**
**dass** eine Zwischenschicht mit einem Isolierfilm und einem Haftmittel des Isolierfilms zwischen dem brechbaren Blisterfilm und der Platte auf und um den Umfangsrand des zerbrechbaren Abschnitts angeordnet ist.

10. Vorrichtung nach einem beliebigen der vorhergehenden Ansprüche,
**dadurch gekennzeichnet,**
**dass** der Träger für mindestens eine Pillenreihe vorgesehen ist, wobei jede Zelle durch Aufreißen von einer Ecke aus abziehbar ist und vieleckige ausgenommene, der Ecke gegenüberliegende Abschnitte aufweist, wobei mindestens eine Polygonspitze mit einem Schneidlinie-Abschnitt zwischen zwei zerbrechbaren Abschnitten für angrenzende Zellen endet, welcher ein Endstück aufweist, das einen Auslasswinkel bildet, der angeordnet ist, um das Aufreißen des Blisters zu initiieren und zu führen.

11. Vorrichtung nach Anspruch 10,
**dadurch gekennzeichnet,**
**dass** der Träger (75) für zwei Pillenreihen vorgesehen ist, bei denen jede Zelle durch Aufreißen von einer Ecke aus abziehbar ist und mittige rautenförmige Ausnehmungen (82) aufweist, die in der Mitte eines jeweiligen Satzes von vier zerbrechbaren Abschnitten für angrenzende Zellen angeordnet sind, damit diese Ecken erreicht werden können, wobei die seitlichen Spitzen (83) der jeweiligen Rauten jeweils mit einem Schneidlinie-Abschnitt (84) zwischen zwei zerbrechbaren Abschnitten für angrenzende Zellen enden, welcher ein Endstück (85) aufweist, das einen Auslasswinkel bildet, der angeordnet ist, um das Aufreißen des Blisters zu initüeren und zu führen.

12. Vorrichtung nach einem beliebigen der Ansprüche 1 bis 10,
**dadurch gekennzeichnet,**
**dass** jeder leitende Abschnitt einen Zweig aufweist, der vollständig außerhalb des zerbrechbaren Abschnitts angeordnet ist, so dass das transparente Lesen des Blisters erleichtert wird.

13. Vorrichtung nach einem beliebigen der vorhergehenden Ansprüche,
**dadurch gekennzeichnet,**
**dass** jeder zerbrechbare Abschnitt (14, 34, 60, 77) eine mittige Zunge (36, 51, 63, 80) aufweist, die an den Seiten durch Ausnehmungsbereiche (35, 50, 71, 81) ausgeschnitten ist, welche angeordnet sind, um gegebenenfalls eine Verschiebung beim Kleben des Blisters auf die Platte zu korrigieren.

14. Vorrichtung nach einem beliebigen der Ansprüche 1 bis 12,
**dadurch gekennzeichnet,**
**dass** jeder zerbrechbare Abschnitt (86) durch eine mittige Zunge (87) gebildet ist, die an den Seiten durch einfache Schneidstriche (88, 89) ausgeschnitten ist, entlang denen Löcher (90) regelmäßig verteilt sind, welche angeordnet sind, um den Auswurf der Pille zu erleichtern.

15. Vorrichtung nach einem beliebigen der vorhergehenden Ansprüche,
**dadurch gekennzeichnet,**
**dass** die Befestigungsmittel (6) durch eine Klebeschicht gebildet sind, die durch einen Kunststofffilm (20) geschützt ist, der abgezogen werden kann, bevor der Blister auf der Platte befestigt wird.

16. Vorrichtung nach einem beliebigen der vorhergehenden Ansprüche,
**dadurch gekennzeichnet,**
**dass** die Platte aus Polyethylennaphtalat (PEN), aus Polyimid oder aus Polyester besteht.

17. Vorrichtung nach einem beliebigen der vorhergehenden Ansprüche,
**dadurch gekennzeichnet,**
**dass** die leitenden Abschnitte, die geeignet sind, rechtwinklig bzw. im Wesentlichen rechtwinklig zu den Pillen des Blisters zu sein und gebrochen zu werden, wenn diese manuell von einem Benutzer ausgeworfen werden, durch Laschen mit zwei abnehmbaren Schenkeln (67) gebildet sind, die durch Bonddrähte (68) über einen mittigen Abschnitt (69) miteinander verbunden werden, wobei die Bonddrähte durch Perforationen geschwächt sind, die hieran in der Platte ausgeführt sind.

18. Vorrichtung nach einem beliebigen der vorhergehenden Ansprüche,
**dadurch gekennzeichnet,**
**dass** n zwischen 5 und 10 liegt und/oder mindestens zwei aufeinander folgende Paare gemittelter Spannungen geprüft werden, um einen Pillenauswurf freizugeben.

## Claims

1. Device (1) for measuring compliance comprising a circuit board (2, 32, 47,73) equipped with an electronic circuit (3, 33) for detecting and recording the moment of extraction of the pills (4, 22, 97) of a blister pack (5) and means (6) for fixing the blister pack to the circuit board, the circuit board being formed of a carrier (7, 75) having the conductor portion (8, 33, 46) of said circuit to form switches (9) designed to be open when the pills are extracted manually by a user, the conductor portion comprising a printed wire of conductor metal, the carrier (7, 75) being formed of a thin, flexible, pliable sheet of plastic of great flexibility, the circuit board comprising breakable portions (14, 34, 60, 77, 86) cut at the sides and distributed uniformly to correspond with a corresponding pill, the circuit having means (16) for measuring the overall impedance of the conductor portion in order to deduce therefrom the moment of extraction of a pill and the wire having identical conducting portions distributed uniformly in parallel along a conductor path, the conductor path being on each extraction so arranged as to exhibit a greater resistance than the portion in parallel,
**characterised in that** the plastic sheet is transparent, **in that** at least four break points (41, 42, 52, 52', 53, 53', 68) in these conducting portions are formed for each breakable portion and corresponding pill, at the edge of said breakable portions, such that the breakable portion of the circuit board and at least one break point of the corresponding conducting portion are broken simultaneously when a pill is extracted,
and **in that** it has means for calculation arranged to retest the impedance regularly, said means comprising means for comparing at least one first mean voltage Tᵢ over n measurements with a preceding mean voltage Tᵢ₋₁ over n measurements.

2. Device according to claim 1, **characterised in that** with at least one conducting portion forming a U-shaped tongue straddling the edge of the breakable portion, said break points are situated wholly distant from the edge of said breakable portion, outside the latter.

3. Device according to claim 2, **characterised in that** the distance of the break points from the edge lies between 1 mm and 3 mm.

4. Device according to any one of the preceding claims, **characterised in that** each conducting portion (37) forms a U-shaped tongue crossing the corresponding breakable portion from one side to the other, the four break points (41, 42) situated at the edge of said breakable portion being formed with two of them level with the bar of the U and the other two level with the tops of the limbs of the U.

5. Device according to any one of claims 2 to 4, **characterised in that** the break points are situated in a zone and level with two orifices situated on either side of the limbs of the U, at least one additional orifice being situated between the limbs of the U.

6. Device according to claim 5, **characterised in that** the orifices are formed by circular holes with a diameter of between 0.5 mm and 2 mm.

7. Device according to any one of the preceding claims, **characterised in that** each breakable portion comprises a pre-cut lozenge (121), delimited by two symmetrical banana-shaped grooved portions (122) terminating in orifices (128, 129, 126, 127) situated a distance d from the periphery.

8. Device according to claim 7, **characterised in that** the ends of the banana-shaped grooves are extended by piercing a plurality of small tangential holes forming a pyramid or arc of a circle the apex of which is formed by an orifice (151), situated between the two limbs of the U.

9. Device according to any one of the preceding claims, **characterised in that** an intermediate layer comprising an insulating film and an adhesive for the insulating film is inserted between the splittable film of the blister pack and the circuit board on and around the peripheral edge of the breakable portion.

10. Device according to any one of the preceding claims, **characterised in that** the carrier is designed for at least one row of pills, each recess being peelable by tearing from one corner and comprising polygonal grooved portions facing said corner, at least one apex of the polygon being terminated by a portion of cut line between two breakable portions for adjacent recesses having an end part forming a pitch angle arranged to start and guide the tearing of the blister pack.

11. Device according to claim 10, **characterised in that** the carrier (75) is designed for two rows of pills each recess of which is peelable by tearing from one corner and comprises diamond-shaped central hollows (82) disposed in the centre of each set of four breakable portions for adjacent recesses for access to these corners, the lateral apexes (83) of each of the diamonds being respectively terminated by a portion (84) of cut line between two breakable portions for adjacent recesses having an end part (85) forming a pitch angle arranged to start and guide the tearing of the blister pack.

12. Device according to any one of claims 1 to 10, **characterised in that** each conducting portion comprises a limb wholly external to the breakable portion, such that transparent reading of the blister pack is facilitated.

13. Device according to any one of the preceding claims, **characterised in that** each breakable portion (14, 34, 60, 77) has a central tongue (36, 51, 63, 80) cut at the sides by grooved zones (35, 50, 71, 81) arranged to correct any offset in the gluing of the blister pack to the circuit board.

14. Device according to any one of the claims 1 to 12, **characterised in that** each breakable portion (86) is formed by a central tongue (87) cut at the sides by simple cut lines (88, 89) along which are uniformly distributed holes (90) arranged to facilitate ejection of the pill.

15. Device according to any one of the preceding claims, **characterised in that** the means (6) for fixing are formed of a layer of glue protected by a peelable plastic film (20) before the blister pack is fixed to the circuit board.

16. Device according to any one of the preceding claims, **characterised in that** the circuit board is made of polyethylene naphthalate (PEN), polyimide or polyester.

17. Device according to any one of the preceding claims, **characterised in that** the conducting portions designed to be at right angles or substantially at right angles with the pills of the blister pack and to be broken when the pills are ejected manually by a user, are formed by feet with two detachable legs (67), connected to one another by junction wires (68) via a central portion (69), said junction wires being weakened by perforations formed in the circuit board level with them.

18. Device according to any one of the preceding claims, **characterised in that** n is between 5 and 10, and/or at least two consecutive pairs of mean voltages are checked to validate the extraction of a pill.
